# EUROPEAN PATENT APPLICATION

(11) **EP 4 275 675 A1**
(43) Date of publication of application: **15.11.2023**
(21) Application number: 22736891.7
(22) Date of filing: 07.01.2022
(51) Int. Cl.: A61K 9/00, A61K 47/38, A61K 47/10, A61K 47/26, A61K 47/02, A61K 31/4725, A61K 31/5377, A61K 31/382, A61K 31/5575, A61P 27/02

(54) **DRUG DELIVERY CONTACT LENS AND OPHTHALMIC PHARMACEUTICAL COMPOSITION**

(30) Priority: 08.01.2021 KR 20210002689
(71) Applicant: Dae Won Pharmaceutical Co., Ltd., Seoul 04808 (KR)
(72) Inventor: JEONG, Ok Chan, Gimhae-si, Gyeongsangnam-do 50835 (KR); CHOI, Du Hyung, Daegu 42278 (KR); LEE, Ah Ram, Incheon 22003 (KR); PARK, Sang Wook, Seoul 04572 (KR)
(74) Representative: Lavoix
(86) International application number: PCT/KR2022/000314
(87) International publication number: WO 2022/149914

(57) **Abstract**

The present disclosure relates to a contact lens for drug delivery with improved drug delivery efficiency and bioavailability. Since the contact lens for drug delivery according to the present disclosure is capable of stably releasing a drug, compared to ophthalmic solutions in the art, bioavailability may be expected to be enhanced by reducing the dosage and frequency of administration. In addition, the contact lens for drug delivery according to the present disclosure may be easily applied to various drugs, has little irritation to the ocular mucosa, and is useful for mass production because of its easy preparation method.

## Description

### Technical Field

The present disclosure relates to a contact lens for drug delivery with improved drug delivery efficiency and bioavailability.

### Background Art

Drugs used to treat ocular diseases include ophthalmic solutions (eye drops) and ophthalmic ointments. For these existing drugs for ocular diseases, only about 7 % of the ophthalmic solutions are effectively used, and the rest remains for about 2 minutes before being discharged into the nasal cavity by tear circulation (Expert opinion on drug delivery 2006 3(2) 275-287). In addition, ophthalmic ointments have an advantage of having longer drug retention time compared to ophthalmic solutions, but due to the nature of the formulation, there are issues of foreign body sensation, irritation due to excipients used, and the possibility of interfering with vision during blinking.

Accordingly, the development of a means of drug delivery having long drug retention time and convenience in management has been required, and as a technology resulting from this demand, contact lenses for drug delivery have been developed. However, currently, contact lenses typically being developed for drug delivery are in a form in which a drug polymer film is placed and used inside the contact lenses, and still have issues. Specifically, in order to mold a film form, a high-concentration polymer needs to be used, but the FDA limits the amount of the polymer used as a drug. Therefore, the selection of polymers that may be used is limited, or a high-concentration polymer may be used only through toxicity tests based on very strict standards. In addition, when a high-concentration polymer is used, there are difficulties in shaping the lens, such as not maintaining the shape of the lens during an application process and not being well adhered to the lens, and for water-soluble polymers, dissolution may cause irritation to the ocular mucosa.

In particular, it is important for contact lenses for drug delivery that the drug is constantly released for a desired period of time. However, polymer film contact lenses have a large area of polymer exposed to the ocular mucosa, and an initial drug release rate rapidly increases due to drug dissolution by tears. In addition, each person has a different amount of tear secretion, which may lead to different patterns of drug release. That is, there is an issue in that the drug release pattern is greatly influenced by the amount of tears.

Accordingly, the inventors of the present disclosure implemented a structure different from existing contact lenses for drug delivery in order to solve the above issues in the art, and such a structure reduces the dosage and frequency of administration as compared to ophthalmic solutions through sustained drug release, and allows stable release of the drug compared to contact lenses for drug delivery in the art, and therefore, a contact lens for drug delivery of the present disclosure was found to be optimized for the treatment of ocular diseases, and the present disclosure was completed.

### Disclosure

### Technical Problem

An aspect is to provide a contact lens, including: a lens main unit; a chamber unit for storing a drug, spaced apart from the center of the lens main unit; and a blocking unit for controlling drug release, bonded to a portion of the chamber unit, wherein the chamber unit includes a channel unit through which a drug moves from the chamber unit and is released to an eye.

Another aspect is to provide an ophthalmic composition including: an ophthalmic pharmacologically active drug; and an excipient for controlling drug release of at least one of a viscous agent, an emulsifier, and a stabilizer.

### Technical Solution

An aspect provides a contact lens, including: a lens main unit; a chamber unit for storing a drug, spaced apart from the center of the lens main unit; and a blocking unit for controlling drug release, bonded to a portion of the chamber unit, wherein the chamber unit includes a channel unit through which a drug moves from the chamber unit and is released to an eye.

The contact lens for drug delivery of the present disclosure, compared to existing ophthalmic solutions and contact lenses for drug delivery, is characterized in that the contact lens is capable of controlling drug release by improving the structure, improving bioavailability, and stably releasing a drug.

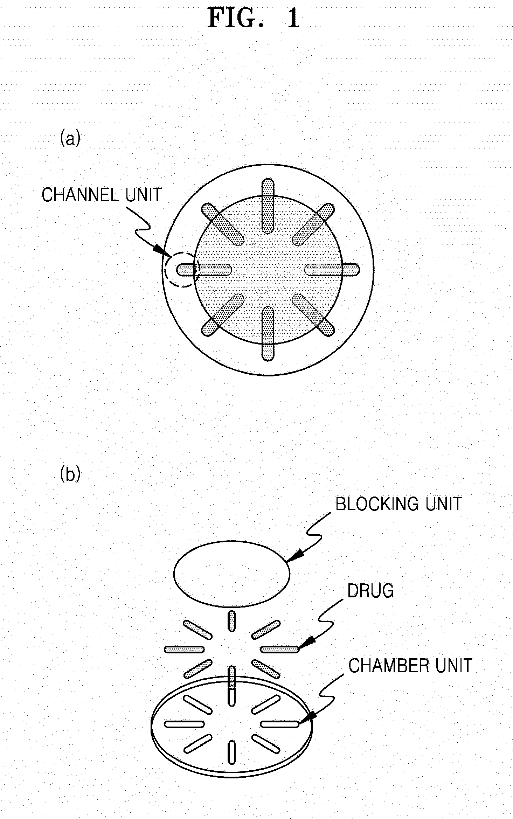

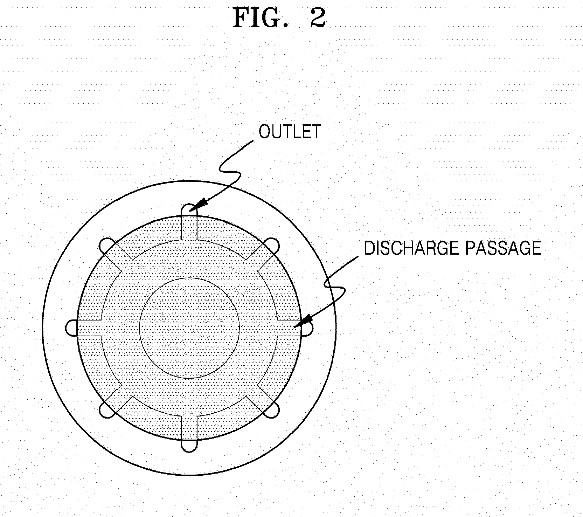

Specifically, a contact lens for drug delivery according to an example of the present disclosure is shown in FIG. 1 or FIG. 2.

Referring to FIG. 1, the contact lens for drug delivery of the present disclosure has a specific shape (for example, straight shape, etc.) inside a lens main unit, and includes: a chamber unit, which is a storage space for a small amount of an ophthalmic pharmacologically active drug; a blocking unit bonded to a portion of the chamber unit; and as a portion of the chamber unit, a channel unit through which the drug moves from the chamber unit and is released into the eye. The channel unit may include an outlet through which the drug is released. An ophthalmic pharmacologically active drug is injected into the chamber unit.

Referring to FIG. 2, the contact lens for drug delivery of the present disclosure may further include a discharge passage in the channel unit through which the drug stored in the chamber unit moves to an outlet. The discharge passage may be applied when finer control of drug release is required. Accordingly, the discharge passage may or may not be included in the contact lens for drug delivery depending on a shape of the chamber unit.

The term "lens main unit", used herein, refers to one having a spherical surface so that it may be worn on the eye. A material of the lens main unit is a generally used material for making a lens, specifically, may be a hydrophilic polymer or a hydrophobic polymer, but is not particularly limited thereto, and may be appropriately selected by those skilled in the art.

The hydrophilic polymer may include at least one selected from the group consisting of acacia, agar, alginic acid, carbomer, carrageenan, cellulose acetate, ceratonia, chitosan, chondroitin sulfate, dermatan sulfate, dextran, ethyl cellulose, gelatin, guar gum, hydroxyethyl cellulose, hydroxypropyl betadex, hydroxypropyl cellulose, hypromellose, hypromellose acetate succinate, hypromellose phthalate, karaya gum, locust bean gum, methyl cellulose, molasses, pectin, polyacrylamide, polycaprolactone, polyethylene oxide, polyethylene glycol, poly hydroxyethyl methacrylate, polyorthoester, polyvinyl alcohol, polyvinylpyrrolidone, sodium alginate, carboxymethyl cellulose, sodium hyaluronate, tragacanth, triethyl citrate, and xanthan gum.

The hydrophobic polymer may include at least one selected from the group consisting of acetyl alcohol, acetyl ester wax, acetyltributyl citrate, aluminum monostearate, carnaubanab, cellulose acetate, cellulose acetate phthalate, dibutyl sebacate, ethyl cellulose, glycerin monostearate, glyceryl behenate, glyceryl monooleate, glyceryl palmitostearate, hydrogenated castor oil, hydrogenated vegetable oil type 1, isopropyl palmitate, polycaprolactone, polyglycolide, polylactic acid, polylactide, polymethacrylate, polyoxyglyceride, shellac, stearic acid, stearyl alcohol, tributyl citrate, white wax, yellow wax, and zein.

More specifically, the hydrophobic polymer may be at least one selected from the group consisting of 2-hydroxyethylmethacrylate (2-HEMA), glycerol methacrylate, silicon hydrogel, and phosphorylcholine, but is not limited thereto.

The lens main unit may additionally include a vision correcting unit (vision correcting lens part), when necessary. The vision correcting unit is located at the center of the lens main unit and is positioned on the cornea to refract light coming in to the pupil to adjust the diopter, and thereby may correct myopia, hyperopia, and the like. The contact lens for drug delivery of the present disclosure not only exhibits an effect of improving, preventing, or treating ophthalmic diseases through drug release, but also may perform vision correction, which is a unique function of the contact lens.

The term "chamber unit", used herein, refers to a portion spaced apart from the center of the lens main unit, and is for storing a drug exposed to the eye, specifically, an ophthalmic pharmacologically active drug. The chamber unit may be formed in a concave structure inside the contact lens. In addition, one or a plurality of the chamber units may be formed in a single contact lens, and the plurality of the chamber units may store the same drug, or a plurality of different drugs.

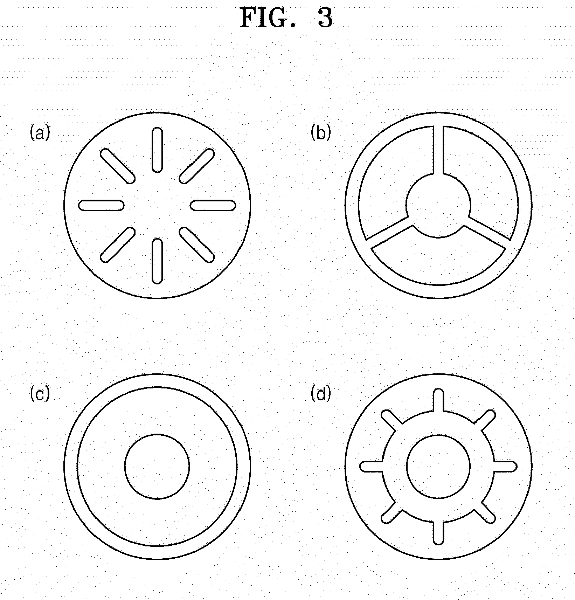

FIG. 3 specifically shows a shape of the chamber unit of the contact lens for drug delivery of the present disclosure. Referring to FIG. 3, the chamber unit may be implemented in a shape of spaces for storing a small amount of drugs in a shape of a plurality of straight lines (see (a) of FIG. 3), spaces for storing three individual drugs (see (b) of FIG. 3), and a space for storing one drug in a large amount (see (c) of FIG. 3), or a fusion type (see (d) of FIG. 3), but is not limited thereto as long as the chamber unit may effectively store drugs.

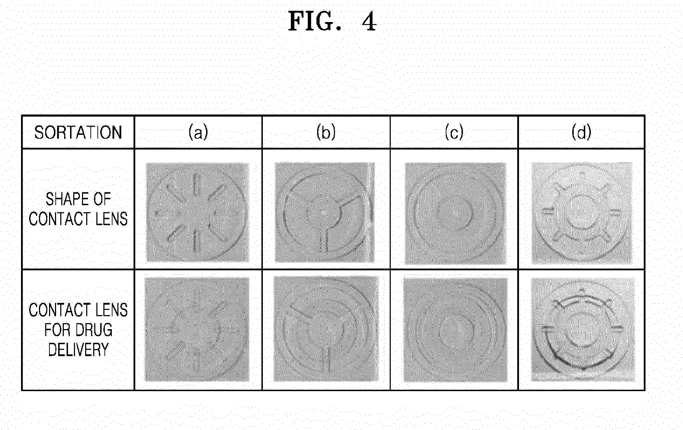

In particular, a shape of the chamber unit may be selected according to an amount of the applied drug, and the size may be adjusted. That the size may be adjusted means a diameter and/or depth of the selected chamber unit, a length of the channel, and the like are changed. That is, a mold for a contact lens may be manufactured by selecting a shape of the chamber unit, and after preparing a contact lens shape by using the same, a drug may be injected and a blocking unit may be bonded to prepare a contact lens for drug delivery (FIG. 4).

The chamber unit may consist of a commonly used material for a lens, and may be, specifically, at least one selected from the group consisting of 2-hydroxyethylmethacrylate (2-HEMA), glycerol methacrylate, silicon hydrogel, and phosphorylcholine, but not limited thereto.

The term, "channel unit", used herein, refers to a region, in which a drug moves to be exposed to the eye, and/or tears, and serves to supply the drug. In this regard, the drug may be slowly released from the channel unit exposed to the eye and/or tears. The channel unit may include an outlet and/or a discharge passage, specifically, may only include an outlet, or include an outlet and a discharge passage.

The term "outlet", used herein, refers to a site where a drug is diacharged (released), and " discharge passage" refers to a passage through which the drug moves from the chamber unit to the channel unit (outlet) in order to be discharged. In this regard, the discharge passage may be such that a drug stored in the chamber unit is released to an eye through the channel unit (outlet) at a constant rate. A length of the discharge passage and a size of the channel unit may be adjusted by a shape of the chamber unit and a size of the blocking unit covering the same, and by adjusting the length of the discharge passage and the size of the channel unit, an amount of the released drug and a rate at which the drug is released may be determined.

The term "blocking unit", used herein, may refer to a portion capable of controlling rates and patterns of drug release. The blocking unit consists of a commonly used material for a lens. The material may be, specifically, at least one selected from 2-hydroxyethylmethacrylate (2-HEMA), glycerol methacrylate, silicon hydrogel, and phosphorylcholine, but is not limited thereto.

The blocking unit may be formed in a size capable of covering 50 area% to 95 area% of the chamber unit, and preferably may be formed in a size capable of covering 70 area% to 95 area% of the chamber unit, but is not limited thereto. Specifically, when the size or blocking ratio of the blocking unit is within the above range, a targeted drug release pattern may be secured.

A blocking ratio of the chamber unit may be controlled according to an area of the chamber unit covered by the blocking unit, and an area% of the chamber unit covered by the blocking unit may mean the blocking ratio of the chamber unit. The blocking ratio of the chamber unit by the blocking unit may be 50 % or more and less than 100 %, specifically, 50 % to 99 %, 50 % to 97 %, 50 % to 95 %, 50 % to 93 %, 60 % to 99 %, 60 % to 97 %, 60 % to 95 %, 60 % to 93 %, 70 % to 99 %, 70 % to 97 %, 70 % to 95 %, 70 % to 93 %, 75 % to 99 %, 75 % to 97 %, 75 % to 95 %, 75 % to 93 %, 80 % to 99 %, 80 % to 97 %, 80 % to 95 %, 80 % to 93 %, 85 % to 99 %, 85 % to 97 %, 85 % to 95 %, 85 % to 93 %, 90 % to 99 %, 90 % to 97 %, 90 % to 95 %, or 90 % to 93 %.

The contact lens for drug delivery may be for releasing a drug at a constant rate. According to an example, the contact lens for drug delivery of the present disclosure may control a release rate of the drug stored in the chamber unit by controlling the blocking ratio of the chamber unit by the blocking unit (area % of the chamber unit covered by the blocking unit), specifically, the contol of the release rate may be adjusting so that the drug stored in the chamber unit is released at a constant rate. Therefore, the contact lens for drug delivery of the present disclosure is characterized in that the drug may be released in a controlled pattern without being released all at once at an initial stage. The controlled pattern includes zero order model, first order model, Higuchi model, Noyes-Whitney model, Korsmeyer-Peppas mode, etc., but is not limited thereto.

The contact lens for drug delivery may be a contact lens for sustained-release drug delivery. The term "sustained release", used herein, means that the drug is slowly released in the body over a long period of time by controlling the release mechanism/pattern of the drug. Specifically, the sustained release may be implemented by controlling the blocking ratio of the chamber unit by using the blocking unit.

An average thickness of the blocking unit may be 30 µm to 80 µm, preferably 40 µm to 60 µm, but is not limited thereto.

The chamber unit may include a drug and an excipient for controlling drug release. The excipient for controlling drug release may include at least one selected from the group consisting of a viscous agent, an emulsifier, a stabilizer, a pH adjusting agent, an isotonic agent, and a preservative.

The viscous agent may include at least one selected from the group consisting of xanthan gum, carbomer, carbopol, polyvinyl alcohol, hydroxyethyl cellulose, hydroxypropyl methylcellulose, methyl cellulose, sodium carboxymethyl cellulose, povidone, polyethylene glycol, polyethylene glycol 400, polyethylene glycol 4000, glycerin, dextran, alginic acid, glucose, dextrose, and propylene glycol dicaprylate, but is not limited thereto.

For a content of the viscous agent, with respect to a total content of an ophthalmic composition including a drug, 0.0015 w/v% to 0.15 w/v% of xanthan gum, 0.005 w/v% to 0.5 w/v% of carbomer, 0.002 w/v% to 0.2 w/v% of carbopol, 0.018 w/v% to 1.8 w/v% of polyvinyl alcohol, 0.005 w/v% to 0.5 w/v% of hydroxyethyl cellulose, 0.02 w/v% to 2.0 w/v% of hydroxypropyl methylcellulose, 0.014 w/v% to 1.4 w/v% of methyl cellulose, 0.005 w/v% to 0.5 w/v% of sodium carboxymethyl cellulose, 0.04 w/v% to 4.0 w/v% of povidone, 0.04 w/v% to 4.0 w/v% of polyethylene glycol, 0.08 w/v% to 8.0 w/v% of polyethylene glycol 400, 0.02 w/v% to 2.0 w/v% of polyethylene glycol 4000, 0.025 w/v% to 2.5 w/v% of glycerin, 0.001 w/v% to 0.1 w/v% of dextran, 0.01 w/v% to 1.0 w/v% of alginic acid, 0.0009 w/v% to 0.09 w/v% of glucose, 0.00065 w/v% to 0.065 w/v% of dextrose, and 0.0005 w/v% to 0.05 w/v% of propylene glycol dicaprylate may be used.

The emulsifier may include at least one selected from the group consisting of polyoxy 40 hardened castor oil, polyoxy 40 hydrogenated castor oil, polyoxyethylene hardened castor oil, polyoxyethylene hardened castor oil 60, polyoxyethylene hydrogenated castor oil, polyoxyl 35 castor oil, propylene glycol, poloxamer, polysorbate 80, tyloxapol, polyethylene glycol 4000, povidone, macrogolglycerol hydroxystearate, and castor oil, but is not limited thereto.

For a content of the emulsifier, with respect to a total content of an ophthalmic composition including a drug, 0.0005 w/v% to 0.05 w/v% of polyoxy 40 hardened castor oil, 0.002 w/v% to 0.2 w/v% of polyoxy 40 hydrogenated castor oil, 0.001 w/v% to 0.1 w/v% of polyoxyethylene hardened castor oil, 0.003 w/v% to 0.3 w/v% of polyoxyethylene hardened castor oil 60, 0.001 w/v% to 0.1 w/v% of polyoxyethylene hydrogenated castor oil, 0.05 w/v% to 5.0 w/v% of polyoxyl 35 castor oil, 0.03 w/v% to 3.0 w/v% of propylene glycol, 0.0005 w/v% to 0.05 w/v% of poloxamer, 0.01 w/v% to 1.0 w/v% of polysorbate 80, 0.005 w/v% to 0.5 w/v% of tyloxapol, 0.02 w/v% to 2.0 w/v% of polyethylene glycol 4000, 0.04 w/v% to 4.0 w/v% of povidone, 0.05 w/v% to 5.0 w/v% of macrogolglycerol hydroxystearate, and 0.0126 w/v% to 1.26 w/v% of castor oil may be used.

The stabilizer may include at least one selected from the group consisting of sodium edetate hydrate, sodium citrate, povidone, polyvinyl alcohol, hydroxyethyl cellulose, methyl cellulose, hydroxypropyl methylcellulose, polysorbate 80, tyloxapol, tromethamine, propylene glycol, sodium hydrogen sulfite , dibutylhydroxytoluene, sodium thiosulfate, hydroxypropyl gamma dex, and aminocaproic acid, but is not limited thereto.

For a content of the stabilizer, with respect to a total content of an ophthalmic composition including a drug, 0.005 w/v% to 0.5 w/v% of sodium edetate hydrate, 0.00294 w/v% to 0.294 w/v% of sodium citrate, 0.04 w/v% to 4.0 w/v% of povidone, 0.018 w/v% to 1.8 w/v% of polyvinyl alcohol, 0.005 w/v% to 0.5 w/v% of hydroxyethyl cellulose, 0.014 w/v% to 1.4 w/v% of methyl cellulose, 0.02 w/v% to 2.0 w/v% of hydroxypropyl methylcellulose, 0.01 w/v% to 1.0 w/v% of polysorbate 80, 0.005 w/v% to 0.5 w/v% of tyloxapol, 0.006 w/v% to 0.6 w/v% of tromethamine, 0.03 w/v% to 3.0 w/v% of propylene glycol, 0.001 w/v% to 0.1 w/v% of sodium hydrogen sulfite, 0.00005 w/v% to 0.005 w/v% of dibutylhydroxytoluene, 0.002 w/v% to 0.2 w/v% of sodium thiosulfate, 0.015 w/v% to 1.5 w/v% of hydroxypropyl gamma dex, and 0.002 w/v% to 0.2 w/v% of aminocaproic acid may be used.

The pH adjusting agent may include at least one selected from the group consisting of hydrochloric acid, phosphoric acid, anhydrous sodium hydrogen phosphate, sodium sulfate, citric acid, sodium citrate, acetic anhydride, sodium acetate, sodium hydrogen carbonate, sodium hydroxide, monoethanolamine, borax, boric acid, dry sodium sulfite, sodium metabisulfite, sodium borate, and sodium lactate, but is not limited thereto.

For a content of the pH adjusting agent, with respect to a total content of an ophthalmic composition including a drug, 0.0027 w/v% to 0.27 w/v% of hydrochloric acid, 0.00288 w/v% to 0.288 w/v% of phosphoric acid, 0.00474 w/v% to 0.474 w/v% of anhydrous sodium hydrogen phosphate, 0.012 w/v% to 1.2 w/v% of sodium sulfate, 0.00005 w/v% to 0.005 w/v% of citric acid, 0.00294 w/v% to 0.294 w/v% of sodium citrate, 0.00007 w/v% to 0.007 w/v% of acetic anhydride, 0.029 w/v% to 2.9 w/v% of sodium acetate, 0.0005 w/v% to 0.05 w/v% of sodium hydrogen carbonate, 0.00716 w/v% to 0.716 w/v% of sodium hydroxide, 0.00052 w/v% to 0.052 w/v% of monoethanolamine, 0.0011 w/v% to 1.1 w/v% of borax, 0.02 w/v% to 2.0 w/v% of boric acid, 0.002 w/v% to 0.2 w/v% of dry sodium sulfite, 0.004 w/v% to 0.4 w/v% of sodium metabisulfite, 0.00019 w/v% to 0.019 w/v% of sodium borate, and 0.0002 w/v% to 0.02 w/v% of sodium lactate may be used.

The isotonic agent may include at least one selected from the group consisting of dextrose, glycerin, concentrated glycerin, mannitol, potassium chloride, sodium chloride, calcium chloride hydrate, magnesium chloride hydrate, D-sorbitol, D-sorbitol solution, glucose and propylene glycol, but is not limited thereto.

For a content of the isotonic agent, with respect to a total content of an ophthalmic composition including a drug, 0.00065 w/v% to 0.065 w/v% of dextrose, 0.025 w/v% to 2.5 w/v% of glycerin, 0.026 w/v% to 2.6 w/v% of concentrated glycerin, 0.05 w/v% to 5.0 w/v% of mannitol, 0.0037 w/v% to 0.37 w/v% of potassium chloride, 0.0655 w/v% to 6.55 w/v% of sodium chloride, 0.00048 w/v% to 0.048 w/v% of calcium chloride hydrate, 0.0003 w/v% to 0.03 w/v% of magnesium chloride hydrate, 0.05 w/v% to 5.0 w/v% of D-sorbitol, 0.066 w/v% to 6.6 w/v% of D-sorbitol solution, 0.0009 w/v% to 0.09 w/v% of glucose, and 0.03 w/v% to 3.0 w/v% of propylene glycol may be used.

The preservative may include at least one selected from the group consisting of benzethonium chloride, methyl paraoxybenzoate, propyl paraoxybenzoate, chlorobutanol, benzalkonium chloride, and chlorocresol, but is not limited thereto.

For a content of the preservative, with respect to a total content of an ophthalmic composition including a drug, 0.002 w/v% to 0.1 w/v% of benzethonium chloride, 0.0005 w/v% to 0.05 w/v% of methyl paraoxybenzoate, 0.0005 w/v% to 0.05 w/v% of propyl paraoxybenzoate, 0.25 w/v% to 0.05 w/v% of chlorobutanol, 0.002 w/v% to 0.1 w/v% of benzalkonium chloride, and 0.0005 w/v% to 0.05 w/v% of chlorocresol may be used.

According to an example, the contact lens for drug delivery of the present disclosure is capable of controling a release rate of a drug stored in a chamber unit by controlling a blocking ratio of the chamber unit (area% of the chamber unit covered by the blocking unit), and a combination of compositions/ratios of drug release-controlling excipients. Specifically, the control of the release rate may be controlling so that the drug stored in the chamber unit is released at a constant rate. Therefore, the contact lens for drug delivery of the present disclosure is characterized in that the drug is not released all at once at an initial stage, and the drug may be released at a constant rate.

The contact lens for drug delivery may be a contact lens for sustained-release drug delivery, and specifically, the sustained-release may be implemented by controling a blocking ratio of the chamber unit by the blocking unit, and a combination of compositions/rates of drug release-controlling excipients.

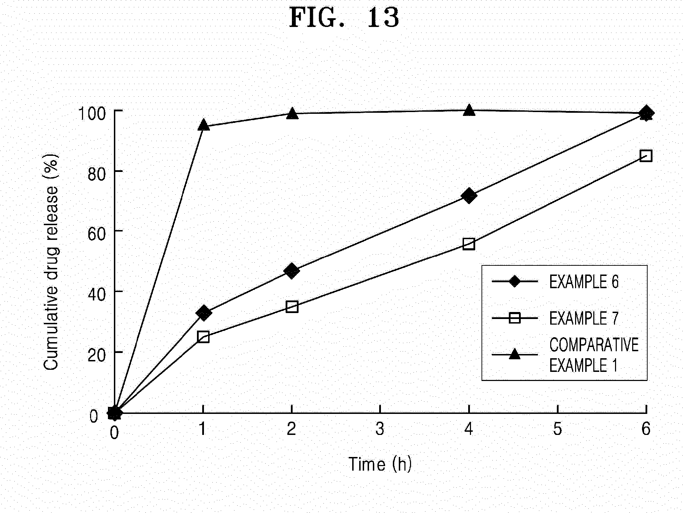

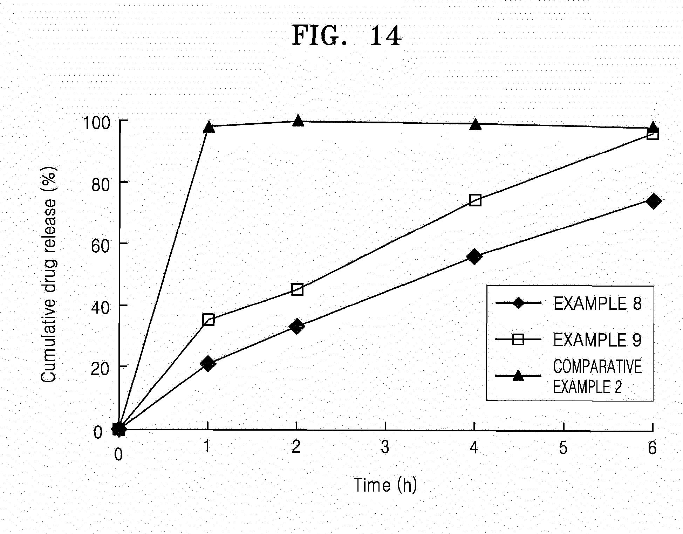

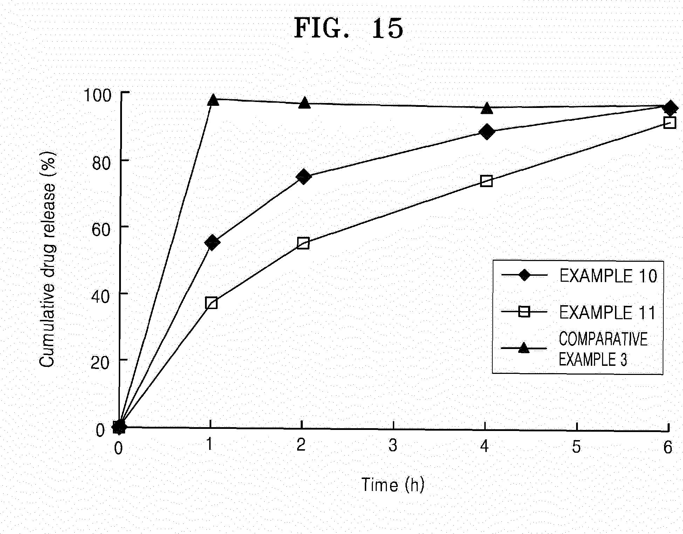

The contact lens for drug delivery may effectively implement release rate control and/or a sustained release effect compared to an ophthalmic solution, specifically, the contact lens for drug delivery may constantly release a drug more effectively than an ophthalmic solution, and therefore, the contact lens may show a more effective therapeutic efficacy with a smaller amount of a drug compared to an ophthalmic solution. For example, the contact lens for drug delivery may exhibit more effective therapeutic efficacy even with a drug in an amount of 0.5 wt% to 50 wt% compared to an ophthalmic solution, specifically, may exhibit an effective therapeutic efficacy with a drug in an amount of 0.5 wt% to 40 wt%, 0.5 wt% to 30 wt%, 0.5 wt% to 20 wt%, 0.5 wt% to 10 wt%, 0.5 wt% to 8 wt%, 0.5 wt% to 6 wt%, 0.5 wt% to 4 wt%, 0.5 wt% to 2 wt%, 1 wt% to 40 wt%, 1 wt% to 30 wt%, 1 wt% to 20 wt%, 1 wt% to 10 wt%, 1 wt% to 8 wt%, 1 wt% to 6 wt%, 1 wt% to 4 wt%, 1 wt% to 2 wt%, 2 wt% to 40 wt%, 2 wt% to 30 wt%, 2 wt% to 20 wt%, 2 wt% to 10 wt% %, 2 wt% to 8 wt%, 2 wt% to 6 wt%, or 2 wt% to 4 wt% compared to an ophthalmic solution, but is not limited thereto. According to an example, as a result of performing an evaluation on drug release by using the contact lens formulation for drug delivery of the present disclosure, and an ophthalmic solution, both including the same/similar composition containing a drug of the same/similar composition, the contact lens formulation was confirmed to show significantly excellent drug release results (drug release at a constant rate) than the ophthalmic solution (Tables 7 to 9, and FIGS. 13 to 15), and it may be seen that when using the contact lens for drug delivery of the present disclosure, a drug may be effectively delivered at a constant rate.

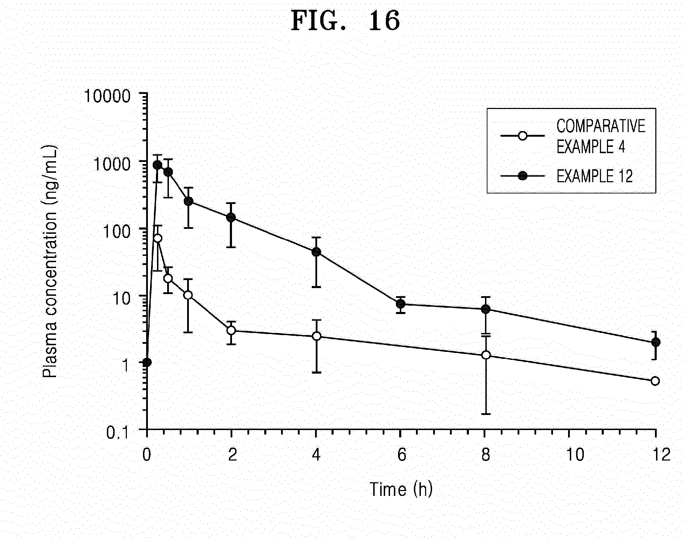

In addition, according to an example, as a result of performing an evaluation of drug administration in the blood and ocular tissues by using the contact lens formulation for drug delivery of the present disclosure, and an ophthalmic solution, both including a drug of the same/similar composition, it was confirmed that bioavailability in the blood, cornea, conjunctiva, sclera, or aqueous humor when using the contact lens formulation for drug delivery increased about 14 times, 37 times, 11 times, 11 times, or 133 times, respectively, compared to the ophthalmic solution (Tables 10 to 14, and FIGS. 16 to 20). Therefore, when using the contact lens formulation for drug delivery of the present disclosure, it may be seen that even with an amount of 0.5 wt% to 10 wt% (blood: about 7.1%, cornea: about 2.7%, conjunctiva: about 9.1%, sclera: about 9.1%, or aqueous humor: about 0.75%) of the drug compared to the ophthalmic solution, an effective therapeutic efficacy may be exhibited.

The drug may be an ophthalmic pharmacologically active drug.

The drug may be composed of at least one selected from the group consisting of low molecular weight compounds, high molecularweight compounds, peptides, and polypeptides, but is not limited thereto.

The drug may include at least one selected from the group consisting of a therapeutic agent for dry eye syndrome, a therapeutic agent for glaucoma, an intraocular pressure reducer, a therapeutic agent for impaired vision, an antibacterial agent, a therapeutic agent for allergic conjunctivitis, a therapeutic agent for blepharoconjunctivitis, a therapeutic agent for night blindness, a therapeutic agent for amblyopia, a therapeutic agent for ocular inflammation, a therapeutic agent for cataract, an antiviral drug, an iridodilator, a carbonic anhydrase inhibitor, and a therapeutic agent for macular degeneration, but is not limited thereto.

The low molecular weight compound may generally refer to a material having a molecular weight of 1,000 or less. The low molecular weight compound may include, but is not limited to, at least one selected from the group consisting of hyaluronic acid in a form of a low molecular weight compound, calcium chloride, sodium chloride, glucose, trehalose, taurine, propylene glycol, cetrimide, asparagine, retinol palmitate, diquafosol, rebamipide, lifitegrast, timolol, dorzolamide, latanoprost, brimonidine, tafluprost, brinzolamide, travoprost, bimatoprost, betaxolol, carteolol, nipradilol, apraclonidine, pilocarpine, levobunolol, isopropyl unoprostone, befunolol, acetazolamide, methazolamide, diclofenamide, unoprostone, verteporfin, levofloxacin, ofloxacin, tobramycin, moxifloxacin, gatifloxacin, oxytetracycline, sulfamethoxazole, glycyrrhizic acid, tosufloxacin, lomefloxacin, chloramphenicol, dexamethasone, tetrahydrozoline, chlorpheniramine, natamycin, ciprofloxacin, enoxolone, fusidic acid, guaiazulene, azulene, erythromycin, gentamicin, sulfamethizole, cefmenoxime, norfloxacin, micronomycin, tetracycline, olopatadine, ketotifen, alcaftadine, bepotastine, azelastine, neostigmine, pyridoxine, epinastine, naphazoline, panthenol, retinol, pheniramine, allantoin, aspartic acid, cyanocobalamin, acitazanolast, cromolyn, tranilast, pemirolast, lodoxamide, N-acetylaspartylglutamic acid, dried bilberry extract, beta-carotene, ascorbic acid, citrulline, tocopherol, riboflavin, fursultiamine, manganese, selenium, ergocalciferol, cefaclor, fluorometholone, tetryzoline, prednisolone, loteprednol, rimexolone, triamcinolone, bromfenac, ketorolac, bendazac, diclofenac, pranoprofen, flurbiprofen, pelubiprofen, neomycin, potassium iodide, sodium iodide, pirenoxine, thiamine, azapentacene, bendazaclysine, nepafenac, acyclovir, ganciclovir, trifluridine, tropicamide, phenylephrine, aminocaproic acid, atropine, cyclopentolate, homatropine, zopolestat, fenofibrate, celecoxib, imrecoxib, polmacoxib, lumiracoxib, etoricoxib, and valdecoxib.

The high molecular weight compound may generally mean a polymer, and may be classified into synthetic polymers and natural polymers. In addition, the high molecular weight compound may refer to a compound, polymer, etc. other than the low molecular weight compounds. A drug in a form of the high molecular weight compound may include carboxymethyl cellulose, hydroxypropyl methylcellulose, polyvinylpyrrolidone, polyacrylic acid, lanolin, dextran, hydroxyethyl cellulose, polysorbate 80, polyvinyl alcohol, polyethylene glycol, carbomer, guar gum, and hydroxymethyl cellulose, but is not limited thereto.

The peptides refer to a polymer in which amino acid units are artificially or naturally linked. The peptides may be classified into dipeptides, tripeptides, oligopeptides, polypeptides, etc. according to a number of amino acid residues. In the present disclosure, the peptide-type drug may consist of one peptide alone, or two or more peptides, specifically, the peptide-type drug may be in a form consisting of one peptide alone, or a form in which two, three, or four peptides are bound. The peptide-type drug may include at least one selected from the group consisting of chondroitin, cyclosporin, ranibizumab, aflibercept, polymyxin B, colistin, bemacizumab, liraglutide, and semaglutide, but is not limited thereto.

For example, the drug may include at least one selected from the group consisting of lifitegrast, timolol, dorzolamide, latanoprost, zopolestat, levofloxacin, pilocarpine, rebamipide, fenofibrate, olopatadine, fluorometholone, pirenoxin, acyclovir, liraglutide, semaglutide, cyclosporine, or a pharmaceutically acceptable salt thereof.

The contact lens for drug delivery may improve a drug penetration rate of the released drug into ocular blood or ocular tissues (cornea, conjunctiva, sclera and aqueous humor), and specifically, may improve a drug penetration rate into ocular blood or ocular tissues (cornea, conjunctiva, sclera and aqueous humor), compared to an ophthalmic solution. Therefore, the contact lens for drug delivery may improve bioavailability of the released drug.

The low molecular weight compound may include, but is not limited to, at least one selected from the group consisting of hyaluronic acid, calcium chloride, sodium chloride, glucose, trehalose, taurine, propylene glycol, cetrimide, asparagine, retinol palmitate, diquafosol, rebamipide, lifitegrast, timolol, dorzolamide, latanoprost, brimonidine, tafluprost, brinzolamide, travoprost, bimatoprost, betaxolol, carteolol, nipradilol, apraclonidine, pilocarpine, levobunolol, isopropyl unoprostone, befunolol, acetazolamide, methazolamide, diclofenamide, unoprostone, verteporfin, levofloxacin, ofloxacin, tobramycin, moxifloxacin, gatifloxacin, oxytetracycline, sulfamethoxazole, glycyrrhizic acid, tosufloxacin, lomefloxacin, chloramphenicol, dexamethasone, tetrahydrozoline, chloramphenicol, dexamethasone, tetrahydrozoline, chlorpheniramine, natamycin, ciprofloxacin, enoxolone, fusidic acid, guaiazulene, azulene, erythromycin, gentamicin, sulfamethizole, cefmenoxime, norfloxacin, micronomycin, tetracycline, olopatadine, ketotifen, alcaftadine, bepotastine, azelastine, neostigmine, pyridoxine, epinastine, naphazoline, panthenol, retinol, pheniramine, allantoin, aspartic acid, cyanocobalamin, acitazanolast, cromolyn, tranilast, pemirolast, lodoxamide, N-acetylaspartylglutamic acid, dried bilberry extract, beta-carotene, ascorbic acid, citrulline, tocopherol, riboflavin, fursultiamine, manganese, selenium, ergocalciferol, cefaclor, fluorometholone, tetryzoline, prednisolone, loteprednol, rimexolone, triamcinolone, bromfenac, ketorolac, bendazac, diclofenac, pranoprofen, flurbiprofen, pelubiprofen, neomycin, potassium iodide, sodium iodide, pirenoxine, thiamine, azapentacene, bendazaclysine, nepafenac, acyclovir, ganciclovir, trifluridine, tropicamide, phenylephrine, aminocaproic acid, atropine, cyclopentolate, homatropine, zopolestat, fenofibrate, celecoxib, imrecoxib, polmacoxib, lumiracoxib, etoricoxib, valdecoxib, carboxymethyl cellulose, hydroxypropyl methylcellulose, polyvinylpyrrolidone, polyacrylic acid, lanolin, dextran, hydroxyethyl cellulose, polysorbate 80, polyvinyl alcohol, polyethylene glycol, carbomer, guar gum, hydroxy methylcellulose, chondroitin, cyclosporine, ranibizumab, aflibercept, polymyxin B, colistin, bevacizumab, liraglutide, semaglutide, and pharmaceutically acceptable salts thereof.

The contact lens for drug delivery may improve a penetration rate of the administered drug into ocular blood or ocular tissues (cornea, conjunctiva, sclera, and aqueous humor), compared to an ophthalmic solution.

According to an example, as a result of evaluating bioavailability of a drug by using the contact lens formulation for drug delivery of the present disclosure, and an ophthalmic solution, both including a drug of the same/similar composition, it was confirmed that bioavailability of the drug and drug concentration retention time were significantly improved when using the contact lens formulation, compared to when using the ophthalmic solution (Tables 10 to 14, and FIGS. 16 to 20), and therefore, it may be seen that when using the contact lens for drug delivery of the present disclosure, the drug may be effectively delivered into the blood vessels and tissues of the eye.

Anther aspect provides an ophthalmic composition, including: an ophthalmic pharmacologically active drug; and at least one excipient for controlling drug release selected from a viscous agent, an emulsifier, and a stabilizer. The same parts as described above also apply to the composition.

The ophthalmic pharmacologically active drug may be included in an amount of 5 w/v% to 20 w/v%, with respect to a total content of a ophthalmic composition, but is not limited thereto.

The ophthalmic composition may further include a pH adjusting agent, an isotonic agent or a preservative.

The ophthalmic composition may be used for improving, preventing, or treating ophthalmic diseases such as, preferably, dry eye syndrome, glaucoma, an increased intraocular pressure, vision impairment, diseases caused by bacteria / virus, allergic conjunctivitis, blepharoconjunctivitis, night blindness, amblyopia, ocular inflammation, cataract, disorders of the pupil, diseases cause by carbonic anhydrase inhibitors, and macular degeneration, but is not limited thereto.

The term "improvement", used herein, means that symptoms of ophthalmic diseases are improved by administration of the ophthalmic composition according to the present disclosure.

The term "prevention", used herein, refers to all acts of suppressing or delaying symptoms of ophthalmic diseases by administering the ophthalmic composition according to the present disclosure.

The term "treatment", used herein, means that symptoms of ophthalmic diseases are beneficially altered by administration of the ophthalmic composition according to the present disclosure.

### [Embodiments]

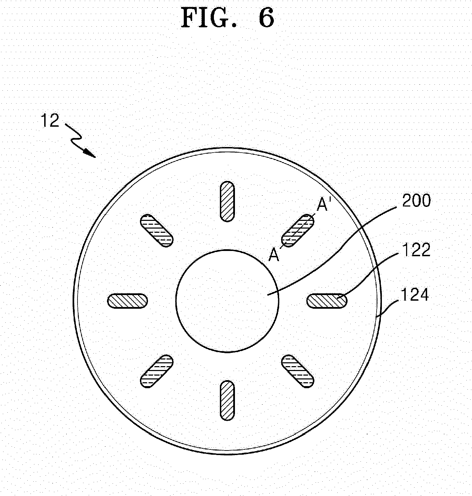

Hereinafter, specific embodiments of the contact lens for drug delivery of the present disclosure will be described with reference to FIGS. 6 to 10. The contact lens according to the present embodiment includes a drug storage structure spaced apart from the center of the contact lens, and is for storing a drug, and provides the drug to the eye of a user of the contact lens. In addition, the contact lens may further include a vision correcting lens part positioned at the center of the contact lens and refracting light.

### Embodiment 1

Hereinafter, an embodiment of the contact lens according to the present disclosure will be described with reference to FIG. 6. However, descriptions of elements identical or similar to those described above may be omitted. FIG. 6 is a cross-sectional view schematically showing an embodiment of the contact lens according to the present disclosure. Referring to FIG. 6, the contact lens includes: a chamber unit 122 formed in a concave structure on the contact lens, and is for storing a drug in the concave structure; a blocking unit 124 covering at least a portion of the chamber unit 122; and a channel unit formed by the blocking unit 124, and is for providing the drug to the eye.

The chamber unit 122 may be formed in a concave structure inside the contact lens 12. For an example, a plurality of chamber units 122 may be formed in a single contact lens 12, and the plurality of chamber units 122 may store a plurality of different drugs. In another example, the plurality of chamber units 122 may store the same drug.

The chamber unit 122 may be formed to have a predetermined volume. For example, the chamber unit 122 may be formed to store a drug in a volume of 1 µl to 2 µl. As another example, the chamber unit 122 may be formed to have a volume corresponding to a volume of a single dose of the stored drug.

As an example of the above embodiment, the blocking unit 124 may be made of silicon. The blocking unit 124 may be formed of any one of the same polymer material as the lens, the hydrophilic polymer material, and the hydrophobic polymer material described above.

### Embodiment 2

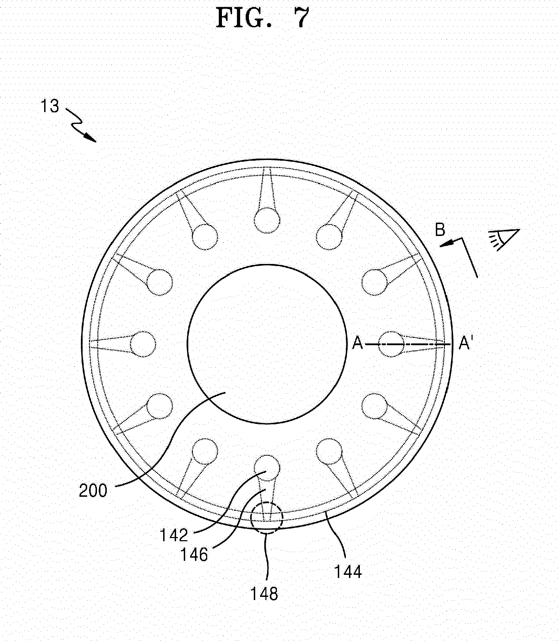

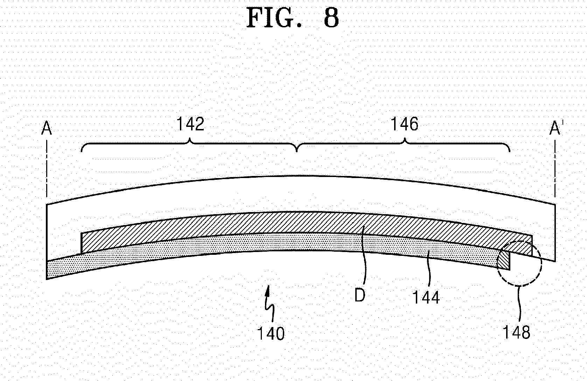

Hereinafter, another embodiment of the contact lens according to the present disclosure will be described with reference to FIGS. 7 to 9. However, descriptions of elements identical or similar to those described above may be omitted. FIG. 7 is a plan view schematically illustrating another embodiment of the contact lens. FIG. 8 is a cross-sectional view schematically illustrating a cross-sectional view taken along a line A-A' of FIG. 7. Referring to FIGS. 7 and 8, the contact lens 13 includes a drug storage structure 140. The drug storage structure 140 includes: a chamber unit 142+146+148 formed in a concave structure to store a drug in the concave structure; a discharge passage 146 through which the drug included in the chamber unit 142+146+148, which is formed in a concave structure to discharge the drug, may move to an outlet; and a blocking unit 144 covering a part of the chamber unit 142+146+148, wherein the blocking unit 144 forms an outlet 148 by opening an end of a drug discharge passage.

The discharge passage 146 is included in the chamber unit 142+146+148 and guides the drug D contained in the chamber unit 142+146+148 to the outlet 148. FIG. 8 illustrates an example in which a discharge passage 146 and a part 142 of the chamber unit are formed at the same height, but in an example not shown, the discharge passage 146 may be formed to have a height smaller than that of the part 142 of the chamber unit.

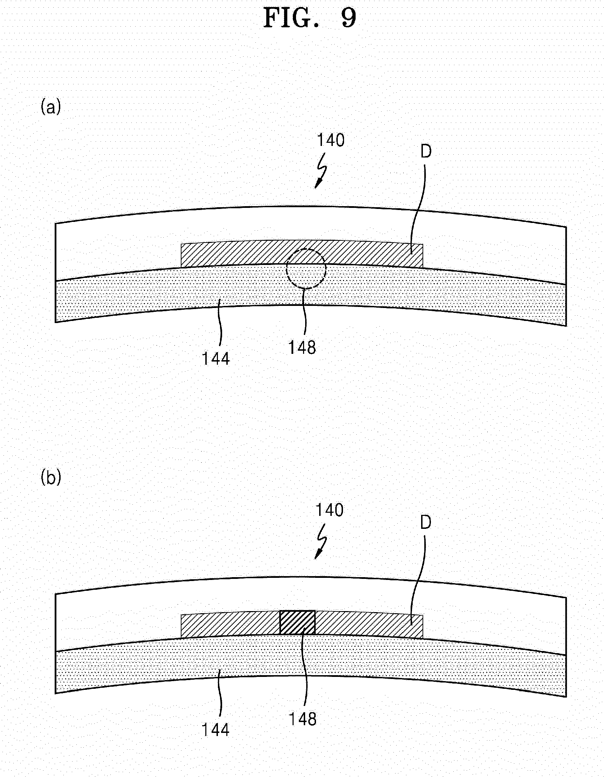

As illustrated in FIG. 7, the blocking unit 144 may be formed to cover a rear surface of the contact lens 13, and may open a lower portion of the discharge passage 146, to form an outlet 148.
(a) of FIG. 9 is a diagram showing an example of the outlet 148, when a cross-section of the contact lens is viewed in direction B of FIG. 7, and (b) of FIG. 9 is a diagram showing another example of the outlet 148. Referring to FIGS. 7 and 8, and (a) of FIG. 9, the blocking unit 144 exposes a lower portion of the discharge passage 146 to form the outlet 148. Thus, according to the example illustrated in (a) of FIG. 9, the outlet 148 discharges the drug D through the lower surface of the contact lens 13.

However, according to the example of the drug outlet illustrated in (b) of FIG. 9, the blocking unit 144 may be formed to cover an entire lower surface of the discharge passage 146, but to open a side end of a discharge passage 146, and thus, as illustrated in (b) of FIG. 9, an outlet 148 may be formed at the end of the discharge passage 146.

People blink their eyelids unconsciously. A user of the illustrated contact lens 13 may unconsciously blink his/her eyelids, and due to the pressure provided by the eyelids moving an upper portion of the contact lens 13, the drug D stored in the chamber unit 142+146+148 may be released through the outlet 148 and provided to the user's eyes.

### Embodiment 3

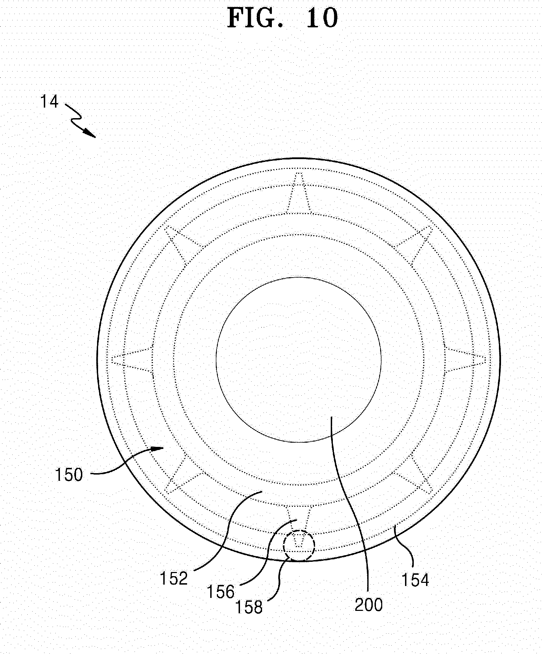

Hereinafter, still another embodiment of the contact lens according to the present disclosure will be described with reference to FIG. 10. However, descriptions of elements identical or similar to those described above may be omitted. FIG. 10 is a plan view schematically illustrating another embodiment of the contact lens. Referring to FIG. 10, the contact lens 14 includes a drug storage structure 150. The drug storage structure 150 includes: a chamber unit 152+156+158 formed in a concave structure to store a drug in the concave structure; a discharge passage 156 included in the chamber unit 152+156+158, which is formed in a concave structure, and is for discharging the drug; and a blocking unit 154 covering a part of the chamber unit 152+156+158, wherein the blocking unit 154 forms an outlet 158 by opening an end of a drug discharge passage.

According to the illustrated example, the chamber unit 152+156+158 may be formed in a circular shape along the center of the contact lens 14. Thus, a single drug may be stored in a large amount.

In the illustrated contact lens 14, the outlet 158 may be formed on the lower surface of the contact lens 14 to discharge the drug D, as in the examples described above, and the outlet 158 may be formed on a side portion of the discharge passage 156 to discharge the drug through an end of the contact lens 14.

### Advantageous Effects

Since a contact lens for drug delivery according to the present disclosure enables constant and continuous release of a drug, bioavailability may be expected to be enhanced by reducing a dosage and frequency of administration compared to an ophthalmic solution in the art, and side effects caused by the dosagemay be reduced, and drug penetration into tissues may be increased. In addition, the contact lens for drug delivery according to the present disclosure may be easily applied to various drugs, has little irritation to the ocular mucosa, and is useful for mass production because of its easy preparation method.

### Description of Drawings

(a) and (b) of FIG. 1 show a structure of a contact lens for drug delivery according to an example of the present disclosure.
FIG. 2 shows a structure of a contact lens for drug delivery according to another example of the present disclosure.
(a) to (d) of FIG. 3 specifically show shapes of a chamber unit of a contact lens for drug delivery of the present disclosure.
(a) to (d) of FIG. 4 show shapes of contact lenses for manufacturing a contact lens for drug delivery according to the present disclosure.
FIG. 5 shows contact lenses for drug delivery according to blocking ratios of a chamber unit by a blocking unit.
FIG. 6 is a plan view schematically illustrating Embodiment 1 of a contact lens for drug delivery according to the present disclosure.
FIG. 7 is a plan view schematically illustrating Embodiment 2 of a contact lens for drug delivery according to the present disclosure.
FIG. 8 is a cross-sectional view schematically illustrating a cross-sectional view taken along a line A-A' of FIG. 7.
(a) of FIG. 9 is a diagram showing an example of an outlet 148 when a cross section of a contact lens is viewed in direction B of FIG. 7, and (b) of FIG. 9 is a diagram showing another example of the outlet 148.
FIG. 10 is a plan view schematically illustrating Embodiment 3 of a contact lens for drug delivery according to the present disclosure.
FIG. 11 shows results of evaluation of a drug release test of a contact lens for drug delivery according to blocking ratios of a chamber portion.
FIGS. 12 to 15 show results of evaluation of drug release tests according to compositions of drug release-controlling excipients included in a chamber unit.
FIG. 16 shows results of evaluation of *in vivo* pharmacokinetics in blood, of a drug released by using a contact lens for drug delivery of the present disclosure.
FIG. 17 shows results of evaluation of *in vivo* pharmacokinetics in the cornea, of a drug released by using a contact lens for drug delivery of the present disclosure.
FIG. 18 shows results of evaluation of *in vivo* pharmacokinetics in the conjunctiva, of a drug released by using a contact lens for drug delivery of the present disclosure.
FIG. 19 shows results of evaluation of *in vivo* pharmacokinetics in the sclera, of a drug released by using a contact lens for drug delivery of the present disclosure.
FIG. 20 shows results of evaluation of *in vivo* pharmacokinetics in the aqueous humor, of a drug released by using a contact lens for drug delivery of the present disclosure.

### Mode for Invention

Hereinafter, the present disclosure will be described in more detail through examples, or experimental examples. However, these examples or experimental examples are intended to illustrate the present disclosure, and the scope of the present disclosure is not limited to these examples.

### Experimental Example 1: Preparation of contact lens for drug delivery including a chamber unit capable of storing drug and a blocking unit, etc.

In order to prepare a contact lens for drug delivery of the present disclosure, the following experiments were performed.

Specifically, molds (female and male) were prepared to manufacture a main unit of the contact lens for drug delivery including a chamber unit of various shapes. The mold was processed by a milling machine (CNC machine 850), using a material of 304-grade stainless steel.

Next, 15 mL of 2-hydroxyethylmethacrylate (2-HEMA) was applied to the female mold, and then the male mold was combined on top of the 2-HEMA to form a contact lens shape. Thereafter, after curing at 80 °C for 1 hour by using a forced convection oven OF-02, the 2-HEMA was separated from the female and male molds, and parts except for the contact lenses were removed to complete the main unit.

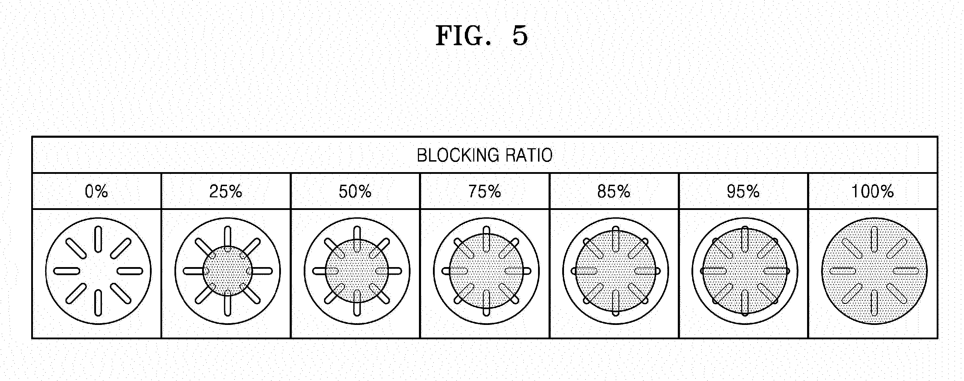

Next, a blocking unit was bonded to an upper part of the main unit. The blocking unit was made of 2-HEMA, and was prepared in various sizes capable of blocking a part or all (25 %, 50 %, 75 %, 85 %, 95 %, 100%) of the chamber area (FIG. 5).

### Experimental Example 2: Evaluation of drug release characteristics according to size of blocking unit (blocking film)

In order to evaluate drug release characteristics according to the size of the blocking unit (blocking film) that is bonded to the chamber unit, which is for storing the drug, and is for blocking the release of the drug stored in the chamber unit, contact lenses for drug delivery of FIG. 4 having various blocking ratios were prepared.

Specifically, contact lenses for drug delivery bonded to blocking units (blocking films) capable of blocking a plurality of straight-shaped chamber units to a ratio of an area of 0 %, 25 %, 50 %, 75 %, 85 %, 95 %, and 100 %, with respect to a total area of the chamber unit were prepared, a drug was injected into each chamber unit, and drug release characteristics were evaluated.

The drug release test was conducted by putting the contact lens for drug delivery into a Franz diffusion cell and evaluating, and the test was conducted while maintaining the temperature at 37.0±0.5 °C and by using a phosphate buffer solution of pH 7.4. A solution in which lifitegrast was dissolved in distilled water was injected into the chamber unit of contact lenses for drug delivery, and the contact lenses were mounted on a donor. In addition, the experiment was conducted in a manner that 5 mL of the solution in which the drug was released was collected with a syringe from each container, and filled with the same amount of buffer solution at a predetermined sampling time (0.5, 1, 1.5, 2, 3, 4, 5, 6 hours), after 5 mL of the solution containing the collected drug was filtered through a syringe filter, 2 mL each was put into a vial and used as a test solution, and the obtained test solution was analyzed by using high-performance liquid chromatography (HPLC).

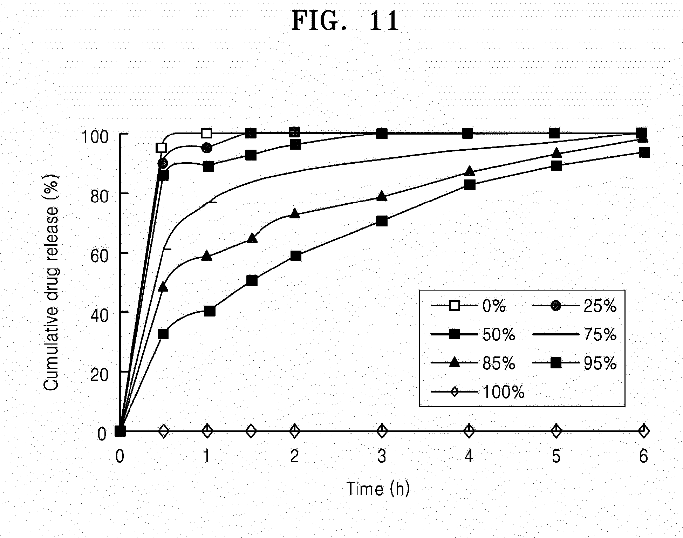

As a result of the above experiment, it was confirmed that drug release patterns differed depending on the size of the blocking unit (blocking ratio of the chamber unit) of the contact lens for drug delivery, specifically, drug release rates were affected by blocking ratios of the chamber units, and it was confirmed that a sustained release effect of the drug was exhibited at a blocking ratio of 50 % to 95 %, but no sustained release effect was observed at a blocking ratio of 100 % (Table 1 and FIG. 11). Based on the above results, in the case of the contact lens for drug delivery of the present disclosure, a certain level of drug release may be acheived by adjusting the blocking ratio of the chamber unit by using the blocking unit (blocking film), and specifically, it may be seen that the drug may be adjusted to be released at a constant rate.

**[Table 1]**

| Time (h) | Cumulative drug release (%) | | | | | | |
|---|---|---|---|---|---|---|---|
| | Blocking ratio | | | | | | |
| | 0 % | 2.5 % | 50 % | 75 % | 85 % | 95 % | 100 % |
| 0 | 0 | 0 | 0 | 0 | 0 | 0 | 0 |
| 0.5 | 95 | 90 | 86 | 61 | 49 | 33 | 0 |
| 1 | 100 | 95 | 90 | 77 | 59 | 41 | 0 |
| 1.5 | 100 | 100 | 93 | 83 | 65 | 51 | 0 |
| 2 | 100 | 100 | 96 | 87 | 73 | 59 | 0 |
| 3 | 100 | 100 | 100 | 91 | 79 | 71 | 0 |
| 4 | 100 | 100 | 100 | 94 | 87 | 83 | 0 |
| 5 | 100 | 100 | 100 | 97 | 93 | 90 | 0 |
| 6 | 100 | 100 | 100 | 100 | 98 | 94 | 0 |

### Experimental Example 3: Evaluation of drug release control when using drug release-controlling excipients

### 3-1: Evaluation of drug release control 1

In the contact lens for drug delivery of the present disclosure, in order to confirm whether releasing of the drug injected into the chamber unit may be controlled by using drug release-controlling excipients, the following experiment was performed.

Specifically, an excipient for controlling drug release was injected together with a drug into the chamber unit, which is a drug reservoir of the contact lens for drug delivery. For the chamber, a shape shown in (a) of FIG. 3 was used, and a blocking unit (blocking film) having a size capable of blocking 75 % of a total area of the chamber unit was used.

A viscous agent, an emulsifier, and/or a stabilizer were used as excipients for controlling drug release. Hydroxypropyl methylcellulose (HPMC) was used as the viscous agent, Poloxamer 188 was used as the emulsifier, and polysorbate 80 was used as the stabilizer. After preparing samples in the ratios shown in Table 2 below, a drug release test was performed.

**[Table 2]**

| Ingredient | | Amount (unit, w/v%) | | | | |
|---|---|---|---|---|---|---|
| | | Examp le 1 | Examp le 2 | Examp le 3 | Examp le 4 | Examp le 5 |
| Main ingredient | Lifitegrast | 10 | 10 | 10 | 10 | 10 |
| Viscous agent | HPMC | - | 1 | - | - | 1 |
| Emulsifier | Poloxamer | - | - | - | 0.05 | 0.05 |
| Stabilizer | Polysorbate 80 | - | - | 1 | - | 1 |
| | Sodium thiosulfate | 0.2 | 0.2 | 0.2 | 0.2 | 0.2 |
| Isotonic agent | Sodium chloride | 5 | 5 | 5 | 5 | 5 |
| pH adjusting agent | Sodium hydroxide | 0.1 | 0.1 | 0.1 | 0.1 | 0.1 |
| | Hydrochloric acid | 0.1 | 0.1 | 0.1 | 0.1 | 0.1 |
| Preservat ive | Benzalkonium chloride | 0.05 | 0.05 | 0.05 | 0.05 | 0.05 |
| Purified water | | Appro priate amoun t | Appro priate amoun t | Appro priate amoun t | Appro priate amoun t | Appro priate amoun t |

The drug release test was conducted by putting the contact lens for drug delivery into a Franz diffusion cell and evaluating, and the test was conducted while maintaining the temperature at 37.0±0.5 °C and by using a phosphate buffer solution of pH 7.4. A composition solution including lifitegrast and drug release-controlling excipients were injected into a chamber unit of a contact lens for drug delivery, and the contact lens was mounted on a donor to proceed the experiment. In addition, the experiment was conducted in a manner that 5 mL of the solution in which the drug was released was collected with a syringe from each container, and the container was filled with the same amount of buffer solution at a predetermined sampling time (0.5, 1, 1.5, 2, 3, 4, 5, 6 hours), after 5 mL of the solution containing the collected drug was filtered through a syringe filter, 2 mL each was put into a vial and used as a test solution, and the obtained test solution was analyzed by using HPLC.

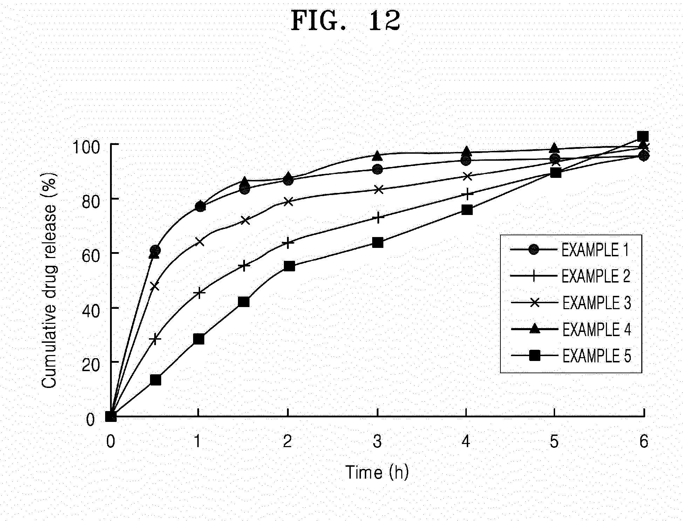

As a result of the above experiment, it was confirmed that a sustained release effect of a drug may be implemented according to the composition and ratio of the viscous agent, emulsifier, and/or stabilizer injected into the chamber of the contact lens for drug delivery (Table 3 and FIG. 12).

**[Table 3]**

| Time (h) | Cumulative drug release (%) | | | | |
|---|---|---|---|---|---|
| | Example 1 | Example 2 | Example 3 | Example 4 | Example 5 |
| 0 | 0 | 0 | 0 | 0 | 0 |
| 0.5 | 61 | 29 | 48 | 60 | 13 |
| 1 | 77 | 45 | 64 | 78 | 29 |
| 1.5 | 83 | 55 | 72 | 86 | 42 |
| 2 | 87 | 64 | 79 | 88 | 54 |
| 3 | 91 | 74 | 84 | 96 | 64 |
| 4 | 94 | 82 | 88 | 97 | 76 |
| 5 | 95 | 90 | 94 | 99 | 90 |
| 6 | 96 | 97 | 99 | 100 | 103 |

Therefore, based on the above results, it may be seen that a targeted drug release pattern may be achieved by simultaneously applying a blocking ratio of the chamber unit by the blocking unit, and drug release-controlling excipients.

### 3-2: Evaluation of drug release control 2

In order to confirm whether drug release-controlling excipients are capable of controlling drug release for various drugs, the following experiment was performed.

Specifically, excipients for controlling drug release were injected together with various types of drugs into a chamber unit, which is a drug storage unit of the contact lens for drug delivery. The chamber used a shape shown in (d) of FIG. 3, and a blocking unit (blocking film) having a size capable of blocking 92 % of a total area of the chamber was used. The drug release test was performed by the method described in Experimental Example 3-1, sampling was performed at fixed times (1, 2, 4, 6 hours), and the samples were analyzed by using HPLC, and the examples were compared with an ophthalmic solution (comparative example).

Lifitegrast, latanoprost, and liraglutide were used as target drugs, and the drug composition solutions injected into the chamber were composed of the compositions shown in Tables 4 to 6 below.

**[Table 4]**

| Ingredient | | Amount (unit, mg/mL) | | |
|---|---|---|---|---|
| | | Contact lens formulation | | Ophthalmic solution |
| | | Example 6 | Example 7 | Comparative Example 1 |
| Main ingredient | Lifitegrast | 200 | 200 | Ophthalmic solution Xiidra (lifitegrast 50 mg/mL) |
| Viscous agent | Carbomer | 1.1 | 1.1 | |
| | HPMC | 0.5 | 1.5 | |
| Stabilizer | Sodium thiosulfate | 3 | 3 | |
| pH adjusting agent | Disodium phosphate | 3.55 | 3.55 | |
| | Hydrochloric acid | 12 | 12 | |
| | Sodium hydroxide | 1 | 1 | |
| Isotonic agent | Sodium chloride | 4.5 | 4.5 | |
| Solvent | Water for injection | Constant volume | | |

**[Table 5]**

| Ingredient | | Amount (unit, mg/mL) | | |
|---|---|---|---|---|
| | | Contact lens formulation | | Ophthalmic solution |
| | | Example 8 | Example 9 | Comparative Example 2 |
| Main ingredient | Latanoprost | 1 | 1 | Ophthalmic solution Xalatan (latanoprost 50 µg/mL) |
| Viscous agent | Carbomer | 1.1 | 1.1 | |
| | HPMC | 0.5 | 0.1 | |
| Stabilizer | Sodium thiosulfate | 3 | 3 | |
| pH adjusting agent | Disodium phosphate | 3.55 | 3.55 | |
| | Hydrochloric acid | 12 | 12 | |
| | Sodium hydroxide | 1 | 1 | |
| Isotonic agent | Sodium chloride | 4.5 | 4.5 | |
| Solvent | Water for injection | Constant volume | | |

**[Table 6]**

| Ingredient | | Amount (unit, mg/mL) | | |
|---|---|---|---|---|
| | | Contact lens formulation | | Ophthalmic solution |
| | | Example 10 | Example 11 | Comparative Example 3 |
| Main ingredient | Liraglutide | 6 | 6 | 6 |
| Viscous agent | Carbomer | 1.1 | 1.1 | 1.1 |
| | HPMC | 0.5 | 1.5 | 0.5 |
| Stabilizer | Sodium thiosulfate | 3 | 3 | 3 |
| pH adjusting agent | Disodium phosphate | 3.55 | 3.55 | 3.55 |
| | Hydrochloric acid | 12 | 12 | 12 |
| | Sodium hydroxide | 2 | 2 | 2 |
| Isotonic agent | Sodium chloride | 30 | 30 | 30 |
| Solvent | Water for injection | Constant volume | | |

As a result of the above experiment, it was confirmed that the drug release pattern may be controlled according to the ratio of excipients in the drug composition solution also for various types of drugs. In addition, when using the contact lens for drug delivery of the present disclosure, it was confirmed that the drug may be released at a constant rate compared to an ophthalmic solution (Tables 7 to 9 and FIGS. 13 to 15).

**[Table 7]**

| Time (h) | Cumulative drug release (%) | | |
|---|---|---|---|
| | Example 6 | Example 7 | Comparative Example 1 |
| 0 | 0 | 0 | 0 |
| 1 | 33 | 25 | 95 |
| 2 | 47 | 35 | 99 |
| 4 | 72 | 56 | 100 |
| 6 | 99 | 85 | 99 |

**[Table 8]**

| Time (h) | Cumulative drug release (%) | | |
|---|---|---|---|
| | Example 8 | Example 9 | Comparative Example 2 |
| 0 | 0 | 0 | 0 |
| 1 | 21 | 35 | 98 |
| 2 | 33 | 45 | 100 |
| 4 | 56 | 74 | 99 |
| 6 | 75 | 96 | 98 |

**[Table 9]**

| Time (h) | Cumulative drug release (%) | | |
|---|---|---|---|
| | Example 10 | Example 11 | Comparative Example 3 |
| 0 | 0 | 0 | 0 |
| 1 | 55 | 37 | 98 |
| 2 | 75 | 55 | 97 |
| 4 | 89 | 74 | 96 |
| 6 | 97 | 92 | 97 |

Therefore, based on the above results, it may be seen that a targeted drug release pattern may be achieved by simultaneously applying a blocking ratio of the chamber unit by the blocking unit, and drug release-controlling excipients.

### Experimental Example 4: Bioavailability improvement test

In order to evaluate *in vivo* pharmacokinetics of a drug released by using the contact lens for drug delivery of the present disclosure, the following experiments were performed.

Specifically, about 6-month-old rabbits capable of wearing contact lenses were used as test animals, and the test animals were subjected to an adaptation period at a breeding condition of 23 ± 2 °C, a humidity of 50 ± 10%, and 12/12 hour light/dark cycle for 1 week before the administration. A contact lens for drug delivery of a composition of No. 6 of an example of Experimental Example 3-1 was used as an experimental group (Example 12), and an ophthalmic solution Xiidra was administered once as a control group (Comparative Example 4), and the results were compared. In order to confirm distribution of the drug in the cornea, conjunctiva, sclera, and aqueous humor, which are active sites of the drug, rabbits were sacrificed and the eyes were extractedat various times for each formulation after administration. Two subjects were sacrificed at each time point, and a number of ocular tissues was set to four. The cornea, conjunctiva, sclera, etc. were separated from the extracted eye, homogenized in distilled water, and a concentration of the drug in the sample was analyzed by using LC-MS/MS. An area under the curve (AUC) of a concentration-time curve and an average concentration were calculated from the drug concentration in the ocular tissues, and changes in a tissue distribution ratio for a dosage in the experimental group was compared to that of the control group, and evaluated.

As a result of analyzing pharmacokinetics in the blood, it was confirmed that the bioavailability increased 14 times when using the contact lens for drug delivery of the present disclosure (Table 10 and FIG. 16), compared to when using an ophthalmic solution.

**[Table 10]**

| Parameters | Comparative Example 4 | Example 12 |
|---|---|---|
| t_{1/2} (h) | 1.98 | 3.30 |
| Tₘₐₓ (h) | 0.25 | 0.33 |
| Cₘₐₓ (ng/mL) | 67.77 | 909.00 |
| AUCₗₐₛₜ (ng·h/mL) | 45.36 | 980.28 |
| AUCₗₐₛₜ/Dose | 9.07 | 122.54 |
| Relative BA (%) | - | 1,351.05 |

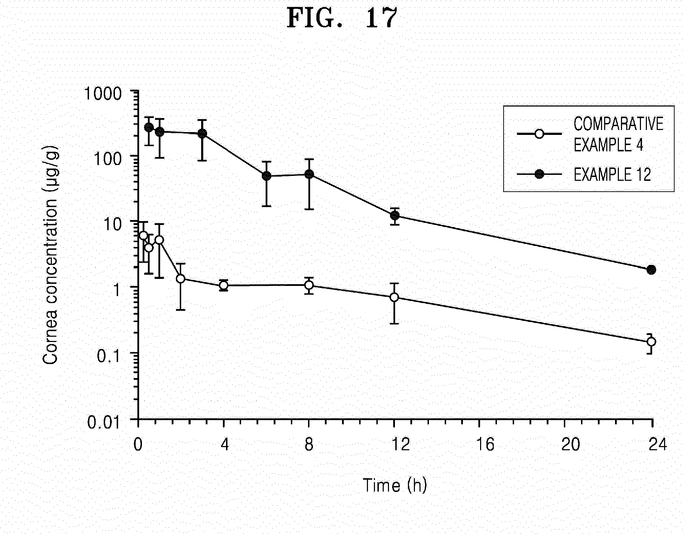

Next, as a result of analyzing the pharmacokinetics in the cornea, it was confirmed that when using the contact lens for drug delivery of the present disclosure, the drug penetration rate into the cornea increased 37 times compared to the control group (ophthalmic solution), and the drug concentration in the cornea was maintained until 3 hours after the administration (Table 11 and FIG. 17).

**[Table 11]**

| Parameters | Comparative Example 4 | Example 12 |
|---|---|---|
| t_{1/2} (h) | 5.38 | 3.90 |
| Tₘₐₓ (h) | 0.25 | 0.50 |
| Cₘₐₓ (µg/mL) | 6.04 | 270.03 |
| AUCₗₐₛₜ (µg·h/mL) | 22.90 | 1351.68 |
| AUCₗₐₛₜ/Dose | 4.58 | 168.96 |
| Relative BA (%) | - | 3,689.08 |

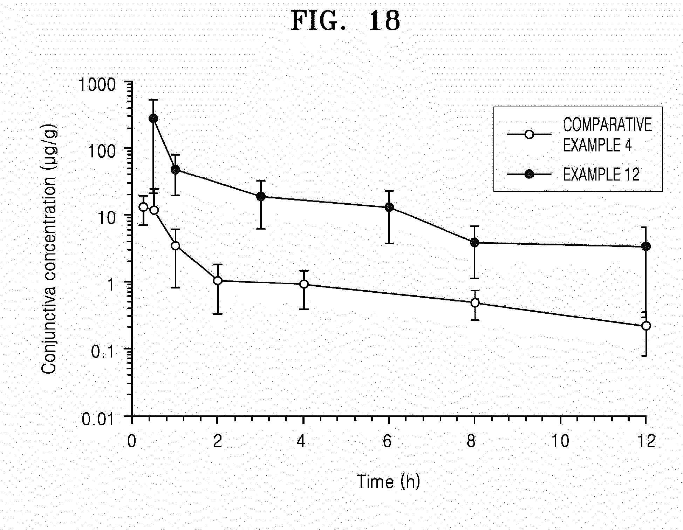

Next, as a result of analyzing the pharmacokinetics in the conjunctiva, it was confirmed that when using the contact lens for drug delivery of the present disclosure, the drug penetration rate into the conjunctiva increased 11 times compared to the control group (ophthalmic solution), and the drug concentration in the conjunctiva was maintained for a long time after the administration (Table 12 and FIG. 18).

**[Table 12]**

| Parameters | Comparative Example 4 | Example 12 |
|---|---|---|
| t_{1/2} (h) | 3.67 | 3.65 |
| Tₘₐₓ (h) | 0.25 | 0.50 |
| Cₘₐₓ (µg/mL) | 13.06 | 278.43 |
| AUCₗₐₛₜ (µg·h/mL) | 17.04 | 299.93 |
| AUCₗₐₛₜ/Dose | 3.41 | 37.49 |
| Relative BA (%) | - | 1,099.41 |

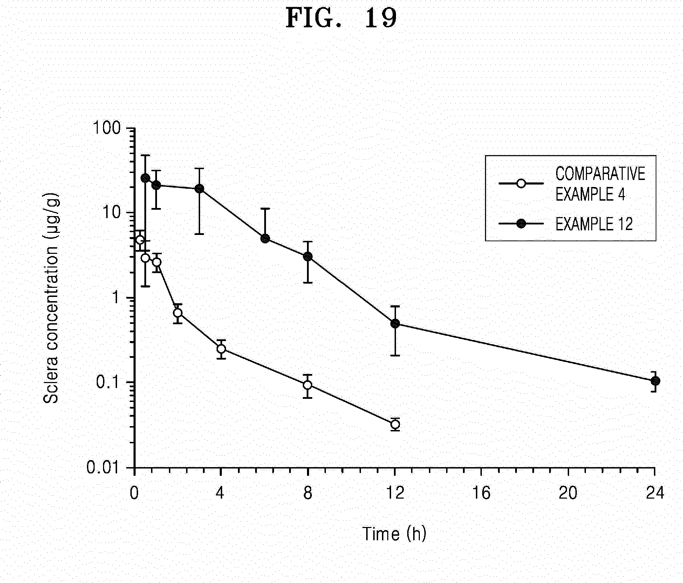

Next, as a result of analyzing the pharmacokinetics in the sclera, it was confirmed that when using the contact lens for drug delivery of the present disclosure, the drug penetration rate into the sclera increased 11 times compared to the control group (ophthalmic solution) (Table 13 and FIG. 19).

**[Table 13]**

| Parameters | Comparative Example 4 | Example 12 |
|---|---|---|
| t_{1/2} (h) | 2.66 | 4.12 |
| Tₘₐₓ (h) | 0.25 | 0.50 |
| Cₘₐₓ (µg/mL) | 4.85 | 25.32 |
| AUCₗₐₛₜ (µg·h/mL) | 6.50 | 114.06 |
| AUCₗₐₛₜ/Dose | 1.30 | 14.26 |
| Relative BA (%) | - | 1,096.92 |

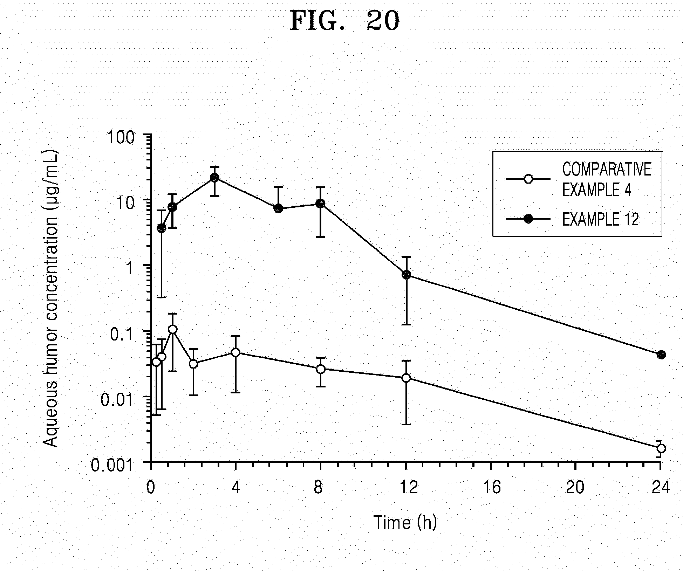

Next, as a result of analyzing the pharmacokinetics in the aqueous humor, it was confirmed that when using the contact lens for drug delivery of the present disclosure, the drug penetration rate into the aqueous humor increased 133 times compared to the control group (ophthalmic solution), and the drug concentration in the aqueous humor was maintained for a long time (Table 14 and FIG. 20).

**[Table 14]**

| Parameters | Comparative Example 4 | Example 12 |
|---|---|---|
| t_{1/2} (h) | 3.81 | 2.75 |
| Tₘₐₓ (h) | 1.00 | 3.00 |
| Cₘₐₓ (µg/mL) | 0.10 | 21.50 |
| AUCₗₐₛₜ (µg·h/mL) | 0.56 | 116.85 |
| AUCₗₐₛₜ/Dose | 0.11 | 14.61 |
| Relative BA (%) | - | 13,281.82 |

To sum up the above results, it may be seen that when using the contact lens for drug delivery of the present disclosure, a penetration rate and bioavailability of drugs in blood and various tissues of the eye (cornea, conjunctiva, sclera, and aqueous humor) may be significantly improved compared to existing ophthalmic solutions, and thus, the contact lens for drug delivery may be effectively used in delivery of ophthalmic pharmacological drugs and treatment/prevention of ophthalmic diseases therethrough.

The above description of the present disclosure is for illustrative purposes, and those skilled in the art to which the present disclosure belongs will be able to understand that the examples and embodiments can be easily modified without changing the technical idea or essential features of the disclosure. Therefore, it should be understood that the above examples are not limitative, but illustrative in all aspects.

### [Explanation of reference numerals]

12, 13, 14: Contact lens
140, 150: Drug storage structure
122, 142+146+148, 152+156+158: Chamber unit
124, 144, 154: Blocking unit
146, 156: Discharge passage
148, 158: Outlet
200: Vision correcting lens part
D: drug

## Claims

1. A contact lens for drug delivery, comprising: a lens main unit;
a chamber unit for storing a drug, spaced apart from the center of the lens main unit; and
a blocking unit for controlling drug release, bonded to a portion of the chamber unit, wherein
the chamber unit comprises a channel unit through which a drug moves from the chamber unit and is released to an eye.

2. The contact lens for drug delivery of claim 1, wherein the channel unit comprises an outlet through which a drug is released.

3. The contact lens for drug delivery of claim 1, wherein the channel unit comprises: an outlet through which a drug is released; and a discharge passage through which a drug moves in order to be released.

4. The contact lens for drug delivery of any one of claims 1 to 3, wherein the blocking unit is formed in a size that is able to cover 50 area% to 95 area% of the chamber unit.

5. The contact lens for drug delivery of any one of claims 1 to 3, wherein the chamber unit comprises a drug and an excipient for controlling drug release.

6. The contact lens for drug delivery of claim 5, wherein the excipient for controlling drug release is at least one selected from the group consisting of a viscous agent, an emulsifier, a stabilizer, a pH adjusting agent, an isotonic agent, and a preservative.

7. The contact lens for drug delivery of any one of claims 1 to 3, wherein the drug is composed of at least one selected from the group consisting of low-molecular-weight compounds, high-molecular-weight compounds, peptides, and polypeptides.

8. The contact lens for drug delivery of any one of claims 1 to 3, wherein the drug comprises at least one selected from hyaluronic acid, calcium chloride, sodium chloride, glucose, trehalose, taurine, propylene glycol, cetrimide, asparagine, retinol palmitate, diquafosol, rebamipide, lifitegrast, timolol, dorzolamide, latanoprost, brimonidine, tafluprost, brinzolamide, travoprost, bimatoprost, betaxolol, carteolol, nipradilol, apraclonidine, pilocarpine, levobunolol, isopropyl unoprostone, befunolol, acetazolamide, methazolamide, diclofenamide, unoprostone, verteporfin, levofloxacin, ofloxacin, tobramycin, moxifloxacin, gatifloxacin, oxytetracycline, sulfamethoxazole, glycyrrhizic acid, tosufloxacin, lomefloxacin, chloramphenicol, dexamethasone, tetrahydrozoline, chlorpheniramine, natamycin, ciprofloxacin, enoxolone, fusidic acid, guaiazulene, azulene, erythromycin, gentamicin, sulfamethizole, cefmenoxime, norfloxacin, micronomycin, tetracycline, olopatadine, ketotifen, alcaftadine, bepotastine, azelastine, neostigmine, pyridoxine, epinastine, naphazoline, panthenol, retinol, pheniramine, allantoin, aspartic acid, cyanocobalamin, acitazanolast, cromolyn, tranilast, pemirolast, lodoxamide, N-acetylaspartylglutamic acid, dried bilberry extract, beta-carotene, ascorbic acid, citrulline, tocopherol, riboflavin, fursultiamine, manganese, selenium, ergocalciferol, cefaclor, fluorometholone, tetryzoline, prednisolone, loteprednol, rimexolone, triamcinolone, bromfenac, ketorolac, bendazac, diclofenac, pranoprofen, flurbiprofen, pelubiprofen, neomycin, potassium iodide, sodium iodide, pirenoxine, thiamine, azapentacene, bendazaclysine, nepafenac, acyclovir, ganciclovir, trifluridine, tropicamide, phenylephrine, aminocaproic acid, atropine, cyclopentolate, homatropine, , zopolestat, fenofibrate, celecoxib, imrecoxib, polmacoxib, lumiracoxib, etoricoxib, valdecoxib, carboxymethyl cellulose, hydroxypropyl methylcellulose, polyvinylpyrrolidone, polyacrylic acid, lanolin, dextran, hydroxyethyl cellulose, polysorbate 80, polyvinyl alcohol, polyethylene glycol, carbomer, guar gum, hydroxymethyl cellulose, chondroitin, cyclosporine, ranibizumab, aflibercept, polymyxin B, colistin, bevacizumab, liraglutide, semaglutide, and pharmaceutically acceptable salts thereof.

9. The contact lens for drug delivery of any one of claims 1 to 3, the contact lens improving a drug penetration rate of a released drug into ocular blood or tissues.

10. The contact lens for drug delivery of any one of claims 1 to 3, further comprising a vision correcting lens part.
